(19) Europäisches Patentamt
European Patent Office
Office européen des brevets

(11) **EP 1 028 318 B1**

(12) **EUROPÄISCHE PATENTSCHRIFT**

(45) Veröffentlichungstag und Bekanntmachung des Hinweises auf die Patenterteilung:
**29.10.2003 Patentblatt 2003/44**

(51) Int Cl.[7]: **G01N 33/72**, G01N 33/49

(21) Anmeldenummer: **00107256.0**

(22) Anmeldetag: **09.07.1997**

(54) **Messanordnung zur optischen Bestimmung der totalen Hämoglobinkonzentration**

Device for the optical measurement of the total hemoglobin concentration

Dispositif pour la détermination optique de la concentration de l'hémoglobine totale

(84) Benannte Vertragsstaaten:
**DE FR GB**

(30) Priorität: **12.07.1996 AT 126296**

(43) Veröffentlichungstag der Anmeldung:
**16.08.2000 Patentblatt 2000/33**

(62) Dokumentnummer(n) der früheren Anmeldung(en) nach Art. 76 EPÜ:
**97890130.4 / 0 818 682**

(73) Patentinhaber: **F.HOFFMANN-LA ROCHE AG**
**4070 Basel (CH)**

(72) Erfinder: **Ziegler, Werner, Dipl.-Ing.**
**8043 Graz (AT)**

(74) Vertreter: **Babeluk, Michael, Dipl.-Ing. Mag.**
**Patentanwalt**
**Mariahilfer Gürtel 39/17**
**1150 Wien (AT)**

(56) Entgegenhaltungen:
EP-A- 0 679 890          US-A- 4 997 769
US-A- 5 061 632          US-A- 5 421 329
US-A- 5 692 503

• **S. TAKATANI ET AL.: "A miniature hybrid reflection type optical sensor for measurement of hemoglobin content and oxygen saturation of whole blood." IEEE TRANSACTIONS ON BIOMEDICAL ENGINEERING., Bd. 35, Nr. 3, März 1988 (1988-03), Seiten 187-198, XP002082099 NEW YORK US**

**Beschreibung**

Die Erfindung betrifft eine Messanordnung zur optischen Bestimmung der totalen Hämoglobinkonzentration in nicht hämolysiertem Vollblut, mit einem Kapillarkanal zur Aufnahme einer Blutprobe, einer Anregungseinrichtung zur Einstrahlung von zumindest zwei Messwellenlängen und einer auf der selben Seite der Kapillare angeordneten Detektionseinrichtung, wobei die optischen Achsen der Anregungseinrichtung und der Detektionseinrichtung auf den Kapillarkanal gerichtet sind, und die Anregungseinrichtung einen durch einen Strahlöffnungswinkel $\alpha$ definierten, von der Anregungseinrichtung ausgehenden Anregungslichtkegel und die Detektionseinrichtung einen durch einen Akzeptanzwinkel $\beta$ definierten, von der Detektionseinrichtung ausgehenden Detektionskegel aufweist.

Es werden folgende Abkürzungen verwendet:

| | |
|---|---|
| Hb | Hämoglobin |
| tHb | totale Hämoglobinkonzentration (Summe aller aktiven und inaktiven Hämoglobinderivate im Blut) |
| $O_{2sat}$ | Sauerstoffsättigung in Prozent |
| $O_2Hb$ | Oxygeniertes Hb |
| RHb | Deoxygeniertes Hb |
| COHb | Carboxy Hb |
| MetHb | Methämoglobin |
| SulfHb | Sulfhämoglobin |
| $\sigma O$ | Absorptionskoeffizient von $O_2Hb$ |
| $\sigma R$ | Absorptionskoeffizient von RHb |
| $\sigma C$ | Absorptionskoeffizient von COHb |
| $\sigma M$ | Absorptionskoeffizient von MetHb |
| $C_O$ | Konzentration von $O_2Hb$ |
| $C_R$ | Konzentration von RHb |
| $C_C$ | Konzentration von COHb |

Eine Messanordnung der eingangs genannten Art ist beispielsweise aus der US-A 5 061 632 bekannt geworden. Das hier beschriebene Oximeter zur Bestimmung von tHb und $O_{2sat}$ weist eine Kapillare zur Aufnahme einer nicht hämolysierten Vollblutprobe auf, welche in die zentrale Bohrung einer zylindrischen Auswerteeinheit eingeführt werden kann. Die Auswerteeinheit weist in axialem Abstand zwei auf die Zentralbohrung im rechten Winkel angeordnete Radialbohrungen auf, über welche die Messstrahlung aus zwei LEDs zugeführt wird. In axialer Erstreckung zwischen den beiden Radialbohrungen für die Lichtzufuhr ist eine weitere senkrecht auf die Zentralbohrung stehende Radialbohrung angeordnet, welche das von der Probe emittierte Licht einem am Mantel der zylindrischen Auswerteeinheit angeordneten Detektor zuführt. Die erste Diode gibt Infrarotstrahlung mit einer Wellenlänge von ca. 800 nm ab und dient zur Bestimmung des tHb. Damit die Messung weitgehend unabhängig von der Verteilung von $O_2Hb$ zu RHb ist, wird die Messung möglichst nahe beim isosbestischen Punkt der Absorptionskoeffizienten von $O_2Hb$ und RHb durchgeführt. Ein derartiger Punkt liegt bei ca. 805 nm (siehe z. B. IEEE Transactions on Biomedical Engineering Vol. 35. No. 3, März 1988). Die zweite Diode emittiert rotes Licht einer Wellenlänge von 660 nm und dient zur Bestimmung der Sauerstoffsättigung $O_{2sat}$, da bei dieser Wellenlänge ein signifikanter Unterschied der Absorptionskoeffizienten für $O_2Hb$ und RHb besteht. Die Beiträge der übrigen Hämoglobinderivate (COHb, MetHb und SulfHb) werden bei der genannten Messanordnung nicht berücksichtigt. Der Fehler für die $O_{2sat}$ Bestimmung liegt dadurch bei ca. 1 bis 15%.

Aus der WO 94/08237 ist ein Verfahren und eine Vorrichtung für direkte spektrofotometrische Messungen in unverdünntem Vollblut beschrieben, bei welchen Messwellenlängen $\lambda_1$ bis $\lambda_n$ in die Probe eingestrahlt und unter einem großen Detektionswinkel erfasst werden. Es wird somit eine Absorptionsmessung unter Berücksichtigung des gestreuten Anteils des eingestrahlten Lichtes bei einer definierten Anzahl von Messwellenlängen durchgeführt. Zur Auswertung muss ein nichtlineares Gleichungssystem gelöst werden, wobei der Anteil der Nichtlinearität von der Probenstärke abhängig ist.

Allgemein sind reproduzierbare Ergebnisse in derartigen Messanordnungen nur dann gewährleistet, wenn keine Veränderungen im optischen Pfad auftreten. Veränderungen werden vor allem durch unterschiedliche Ein- und Auskoppelverluste verursacht, da bei Streulichtmessungen die Weglänge durch die Probe undefiniert ist. Wird nun eine austauschbare Küvette oder Kassette (Einmalsensor) verwendet, ergeben sich nur bedingt reproduzierbare Ein- und Auskoppelfaktoren für das Licht, bedingt durch nicht plane, unterschiedliche Oberflächen der einzelnen Küvetten bzw. Kassetten (Polarisationseffekte, Streuung etc.).

Aus der DD 203 632 A ist ein Schnellverfahren und eine Einrichtung zur fotometrischen Bestimmung der Konzentration von COHb bekannt geworden. Es wird mit zwei Messwellenlängen operiert, wobei als Lichtquellen Luminszenzdioden mit einer maximalen spektralen Emission bei 565 nm und 940 nm verwendet werden. Als Lichtempfänger dienen Festkörperfotodetektoren, welche unmittelbar auf die Probe gerichtet sind, die als mit dem Untersuchungsmaterial getränktes spezielles Filterpapier vorliegt. Zur Bestimmung der spektralen Absorption werden die Remission und/oder die Transmission ausgewertet, wobei die COHb Konzentration über einen Rechner ausgegeben wird.

Aus der AT-E 56 271 B ist ein Verfahren zur spektrofotometrischen Bestimmung der Konzentration einer Anzahl von Hämoglobinderivaten in Vollblut bekannt, wobei mehrere unterschiedliche Wellenlängen in die Blutprobe eingestrahlt und Absorptionswerte gemessen werden. Die Konzentrationen der einzelnen Hämoglobinderivate werden durch Lösen eines Gleichungssystems bestimmt.

Weitere fotometrische Methoden zur Messung von Blutbestandteilen sind aus der US-A 5 127 406 und der US-A 5 064 282 bekannt.

Aufgabe der Erfindung ist es, eine Messanordnung zur optischen Bestimmung der totalen Hämoglobinkonzentration in nicht hämolysiertem Vollblut vorzuschlagen, wobei das Problem der sich ändernden Ein- und Auskoppelverluste gelöst werden soll. Die Messanordnung soll auch zur Bestimmung der Sauerstoffsättigung und für austauschbare Einwegkassetten verwendbar sein.

Diese Aufgabe wird erfindungsgemäß dadurch gelöst, dass die Neigungswinkel der optischen Achsen und deren Abstand an der Schnittstelle mit der Kapillare derart auf den Kapillardurchmesser abgestimmt sind, dass sich der Anregungslichtkegel und der Detektionskegel während einer Kalibriermessung bei mit wässriger Lösung gefülltem Kapillarkanal an der der Anregungseinrichtung und der Detektionseinrichtung gegenüberliegenden Innenwand des Kapillarkanals überlappen und während einer Messung bei mit Vollblut gefülltem Kapillarkanal, bedingt durch die verringerte mittlere Eindringtiefe der Messstrahlung, nicht überlappen, so dass die Messung durch Streuung an den Blutzellen erfolgt. Die Innenwand des Kapillarkanals kann für die verwendeten Messwellenlängen $\lambda_1$, $\lambda_2$ unterschiedliche Reflexionswerte aufweisen, so dass eine Kalibriergröße abgeleitet werden kann, die den Ein- und Auskoppelverlusten proportional ist.

Durch die Überlappung des Anregungslichtkegels und des Detektionskegels bei mit wässriger Lösung, beispielsweise einer Kalibrierlösung, gefülltem Kapillarkanal kann somit eine Leermessung durchgeführt werden, bei welcher das Messlicht, behaftet mit Ein- und Auskoppelverlusten, nach einer Reflexion an der Innenwand des Kapillarkanals in die Detektionseinrichtung gelangt. Durch die Verwendung zumindest zweier Messwellenlängen können die unterschiedlichen optischen Verluste vor jeder Messung erfasst werden. Der Kapillarkanal kann gemäß einer bevorzugten Ausführung der Erfindung in einer Einwegkassette angeordnet sein, welche in ein die Anregungs- und Detektionseinrichtung aufweisendes Auswertegerät einbringbar ist.

Es ist weiters von Vorteil, wenn die Messanordnung eine erste Messwellenlänge mit $\lambda_1 < 805$ nm und eine zweite Messwellenlänge mit $\lambda_2 > 805$ nm bereit stellt, derart, dass für die Absorptionskoeffizienten $\sigma O(\lambda)$ und $\sigma R(\lambda)$ der Hämoglobinderivate $O_2Hb$ und RHb bei den beiden Messwellenlängen $\lambda_1$ und $\lambda_2$ gilt: $\sigma O(\lambda_1)$ ungefähr gleich $\sigma R(\lambda_2)$ und $\sigma R(\lambda_1)$ ungefähr gleich $\sigma O(\lambda_2)$. Es können dann bei den Wellenlängen $\lambda_1$ und $\lambda_2$ die Absorptionswerte $A_1$ und $A_2$ bestimmt werden, wobei die Summe der beiden Absorptionswerte $A_1 + A_2$ eine zur totalen Hämoglobinkonzentration tHb proportionale, von der Sauerstoffsättigung $O_{2sat}$ unabhängige Größe ist.

Die Messanordnung verwendet somit zwei isosbestisch symmetrische Wellenlängen, wobei durch die gewählten Wellenlängen mit herkömmlichen Laserdioden die selben Vorteile erzielt werden, welche eine Messung direkt am isosbestischen Punkt ermöglichen würde. Die Absorptionskoeffizienten von $O_2Hb$ und RHb sollten dabei bei den beiden Wellenlängen $\lambda_1$ und $\lambda_2$ jeweils nur um $\pm 5$ % von einander abweichen. Weiters ist die Messung weitgehend unabhängig von der Sauerstoffsättigung der Probe.

Zur Kompensation unterschiedlicher Anregungsintensitäten $I_1$ und $I_2$ bei den Wellenlängen $\lambda_1$ und $\lambda_2$ kann ein Korrekturfaktor f eingeführt werden, derart, dass die totale Hämoglobinkonzentration tHb der Summe $A_1 + f.A_2$ proportional ist, wobei der Faktor f zwischen 0,5 und 2,0 liegt und eine von der gewählten Messanordnung abhängige Kalibriergröße ist.

Vorteilhafte Messwellenlängen für $\lambda_1$ liegen zwischen 780 und 790 nm, vorzugsweise bei $785 \pm 3$ nm sowie für $\lambda_2$ zwischen 830 und 850 nm, vorzugsweise bei $836 \pm 3$ nm. Für die genannten Wellenlängen sind Standardlaserdioden verfügbar (780 bis 785 nm bzw. 840 bis 850 nm) die eine scharf begrenzte Emissionswellenlänge abstrahlen.

Die Sauerstoffsättigung $O_{2sat}$ kann aus den ermittelten Absorptionswerten $A_1$ und $A_2$ näherungsweise durch die Formel

$$O_{2sat}[\%] = 100.(\sigma R(\lambda_1).A_2 - \sigma R(\lambda_2).A_1)/((A_1 + A_2).(\sigma R(\lambda_1) - \sigma R(\lambda_2)))$$

bestimmt werden. Einer derartigen Bestimmung der Sauerstoffsättigung liegen folgende Überlegungen zugrunde:

$$tHb = C_O + C_R + C_C \quad C_C << C_O + C_R \Rightarrow C_C \sim O$$

$$O_{2sat}[\%] = 100.C_O/(C_O + C_R) \sim 100.C_O/tHb$$

$$A_1 = \sigma R_1.C_R + \sigma O_1 C_O + \sigma C_1.C_C \qquad \text{mit } \sigma C_1.C_C \sim 0$$

$$A_2 = \sigma R_2.C_R + \sigma O_2 C_O + \sigma C_2.C_C \qquad \text{mit } \sigma C_2.C_C \sim 0$$

$$\Rightarrow tHb = [A_1(\sigma R_2-\sigma O_2)+A_2(\sigma O_1-\sigma R_1)]/(\sigma R_2.\sigma O_1-\sigma R_1.\sigma O_2)$$

$$\text{mit } \sigma R_1 \sim \sigma O_2, \sigma R_2 \sim \sigma O_1$$

$$\Rightarrow tHb = (A_1+A_2)/(\sigma R_2+\sigma R_1 = \underline{K.(A_1+A_2)}$$

$$C_O = (A_1\sigma R_2-A_2\sigma R_1)/(\sigma R_2.\sigma O_1-\sigma R_1.\sigma O_2)$$

$$O_{2sat}[\%] = 100. (A_2\sigma R_1-A_1\sigma R_2)/ [(A_1+A_2) (\sigma R_1-\sigma R_2)]$$

Für die Bestimmung der Sauerstoffsättigung kann jedoch auch eine dritte Messwellenlänge verwendet werden, wobei jede Wellenlänge zulässig ist, bei welcher die Absorptionskoeffizienten $\sigma R$ und $\sigma O$ für RHb und $O_2$Hb möglichst große Unterschiede aufweisen. Eine günstige Wellenlänge liegt beispielsweise bei 680 nm (geringer Einfluss von MetHb).

Im Hinblick auf die verwendeten Messwellenlängen ist es von vorteil, dass die Innenwand des Kapillarkanals zumindest im Bereich des sich mit dem Anregungslichtkegel überlappenden Detektionskegel eine rote Kalibrierfläche aufweist. Dabei ist es von Vorteil, wenn die rote Kalibrierfläche einen Mantelbereich der Innenfläche des Kapillarkanals bedeckt, welcher sich über ca. 20 bis 40 % des Umfangs erstreckt und der Anregungs- und Detektionseinrichtung gegenüberliegt.

In einer weiteren Ausgestaltung der Erfindung ist vorgesehen, dass sich der Kapillarkanal im Bereich der sich überlappenden Anregungslicht- bzw. Detektionskegel zu einer Messkammer erweitert, wobei eine Wand der Messkammer einen optischen Sensor aufweist, dessen für den zu messenden Analyten permeable Deckschicht gleichzeitig als rote Kalibrierfläche dient. Zum Beispiel kann der optische Sensor eine Indikatorschicht mit einem Indikator zur Messung des $pO_2$ in der Vollblutprobe aufweisen.

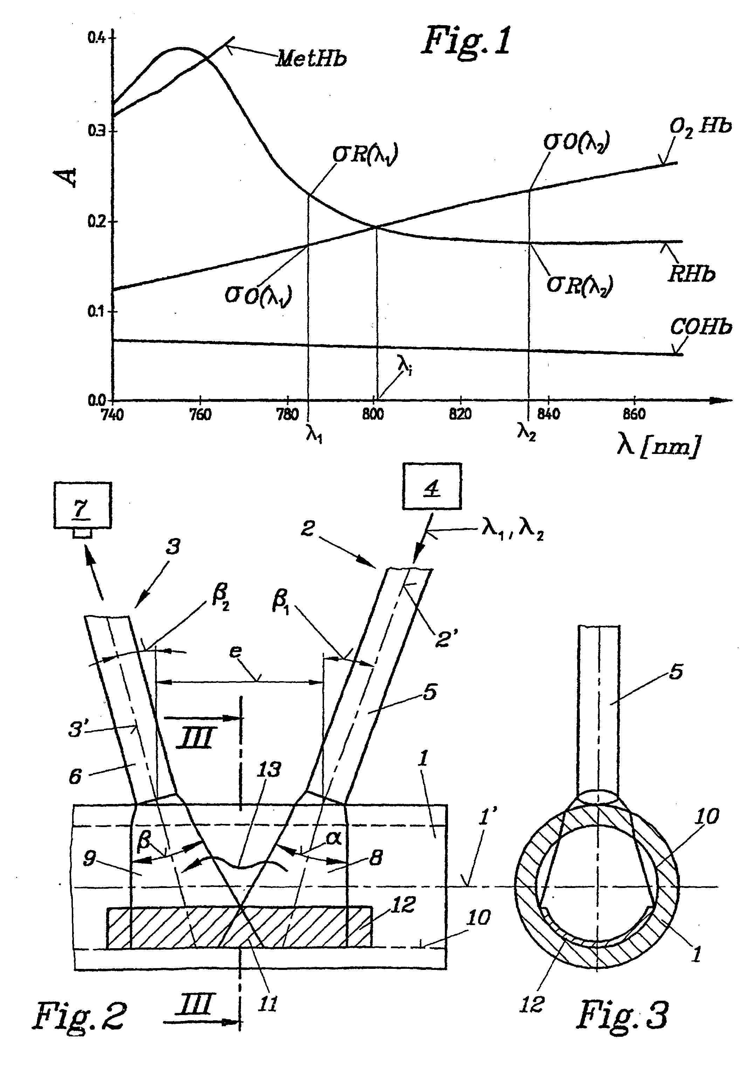

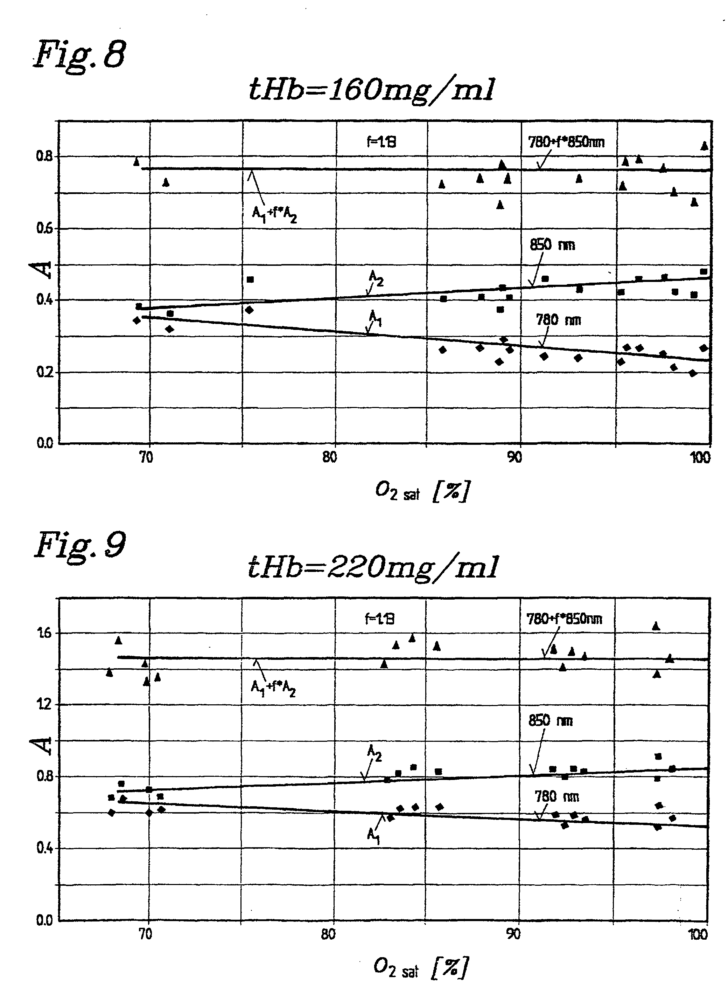

Die Erfindung wird im folgenden anhand von zum Teil schematischen Zeichnungen näher erläutert. Es zeigen Fig. 1 ein Diagramm mit der von der Wellenlänge abhängigen unterschiedlichen Absorption der einzelnen Hämoglobinderivate, Fig. 2 eine erste Ausführungsvariante einer erfindungsgemäßen Messanordnung, Fig. 3 die Messanordnung gemäß Fig. 2, geschnitten entlang der Linie III-III in Fig. 2, die Fig. 4 bis 7 eine weitere Ausführungsvariante der erfindungsgemäßen Messanordnung, sowie Fig. 8 und 9 Diagramme, welche ein von $O_{2sat}$ unabhängiges Summensignal für tHb zeigen.

Aus Fig. 1 sind die unterschiedlichen Beiträge der einzelnen Hämoglobinderivate RHb, $O_2$Hb, COHb und MetHb im Wellenlängenbereich zwischen 740 und 880 nm dargestellt. Ein isosbestischer Punkt für die Hauptbestandteile RHb und $O_2$Hb liegt bei $\lambda_i$ = 805 nm. Aus der Abbildung ist weiters ersichtlich, dass für die eingezeichneten Wellenlängen $\lambda_1$ = 785 nm und $\lambda_2$ = 836 nm $\sigma R (\lambda_1) = \sigma O(\lambda_2)$ und $\sigma O(\lambda_1) = \sigma R(\lambda_2)$ ist. Unter Ausnützung der genannten Bedingung ist die Summe der beiden Absorptionswerte $A_1$ und $A_2$ bei den Wellenlängen $\lambda_1$ und $\lambda_2$ dem tHb der Blutprobe proportional und unabhängig von der Sauerstoffsättigung. Aus Fig. 1 ist weiters ersichtlich, dass der Absorptionskoeffizient von COHb bei beiden Wellenlängen ungefähr gleich groß ist, d. h. $\sigma C(\lambda_1)$ entspricht $\sigma C(\lambda_2)$. Aufgrund des kleinen Wertes des Absorptionskoeffizienten für COHb und der geringen Konzentration von COHb kann deren Produkt für die Berechnung des tHb in erster Näherung vernachlässigt werden.

Die Fig. 2 und 3 zeigen eine erste Ausführungsvariante einer erfindungsgemäßen Messanordnung zur optischen Bestimmung des tHb in nicht hämolysiertem Vollblut. Die Messanordnung weist einen Kapillarkanal 1 zur Aufnahme einer Blutprobe, eine Anregungseinrichtung 2 zur Einstrahlung von zumindest zwei Messwellenlängen $\lambda_1$ und $\lambda_2$ sowie eine Detektionseinrichtung 3 auf, wobei die optischen Achsen 2' und 3' der Anregungseinrichtung 2 und der Detektionseinrichtung 3 auf den Kapillarkanal 1 gerichtet sind. Die Anregungseinrichtung 2 weist eine Einheit 4, welche die zwei Messwellenlängen $\lambda_1$ und $\lambda_2$ zur Verfügung stellt und Lichtleitmittel, z. B. eine Faseroptik 5, zur Lichtzufuhr auf. Desgleichen besteht die Detektionseinrichtung 3 aus einer Faseroptik 6 und einem Detektor 7. Der Anregungseinrichtung 2 ist bedingt durch die numerische Apertur der Faseroptik 5 ein Strahlöffnungswinkel $\alpha$ für den Anregungslichtkegel 8 zugeordnet. Desgleichen weist die Detektionseinrichtung 3 einen durch einen Akzeptanzwinkel $\beta$ definier-

ten Detektionskegel 9 auf.

Beide Kegel 8 und 9 überlappen sich an der Innenwand 10 des Kapillarkanals 1, wenn dieser mit wässriger Lösung gefüllt ist. Um einen genügend großen Überlappungsbereich 11 der beiden Kegel 8 und 9 zu erreichen, kann zumindest eine der Achsen 2' und/oder 3' zur Normale auf die Achse 1' des Kapillarkanals 1 um den Winkel $\beta_1$ und/oder $\beta_2$ geneigt sein. Durch Variation der Entfernung e zwischen der Anregungseinrichtung 2 und der Detektionseinrichtung 3 sowie durch Veränderung der beiden Winkel $\beta_1$ und $\beta_2$ kann die Sensitivität bei der Messung und der Kalibrierung optimiert werden.

Für die Kalibrierung der Messanordnung ist die Innenwand 10 des Kapillarkanals 1 zumindest im Überlappungsbereich 11 der beiden Kegel 8 und 9 mit einer roten Kalibrierfläche 12 versehen. Diese Kalibrierfläche 12 erstreckt sich über ca. 20 bis 40 % des Umfanges der inneren Mantelfläche. Der Innendurchmesser der Kapillare 1 beträgt typischerweise 1 mm.

Nach Abschluss der Kalibrierung wird die Kapillare mit Vollblut gefüllt, wodurch sich die mittlere Eindringtiefe der Messstrahlung auf die obere Hälfte bzw. obere Drittel der Kapillare beschränkt. Die beiden Kegel 8 und 9 überlappen sich dadurch nicht mehr, sodass die Messung durch Streuung an den Blutzellen entlang des Pfeiles 13 erfolgt.

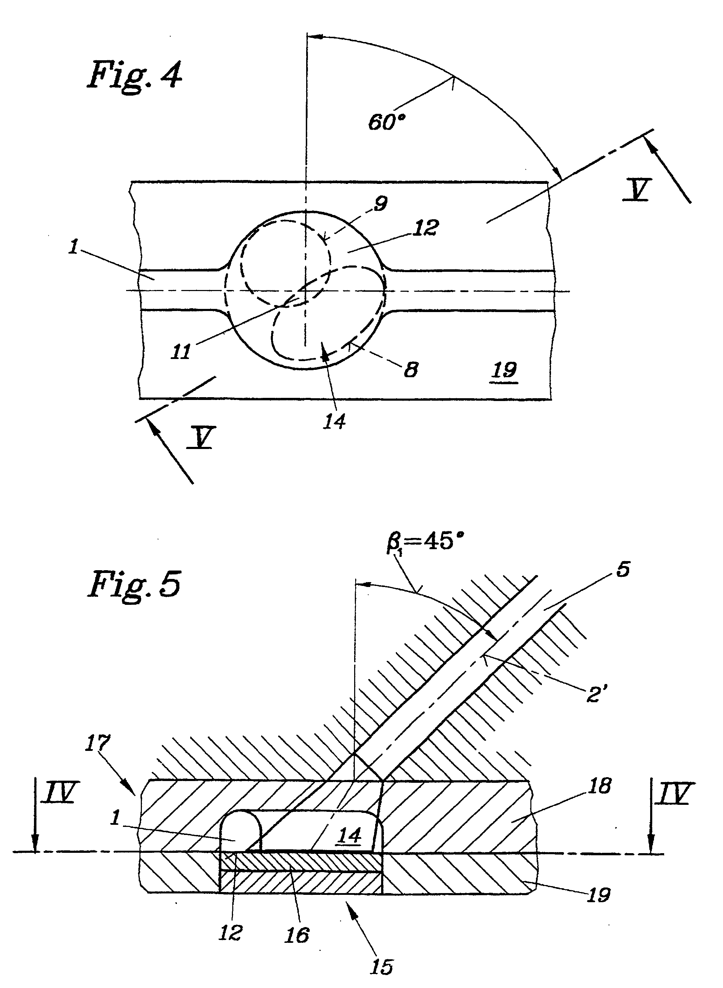

Aus den Fig. 4 und 5 ist eine Ausführungsvariante der Erfindung ersichtlich, bei welcher sich der Kapillarkanal 1 im Bereich 11 der sich überlappenden Anregungslicht- bzw. Detektionskegel 8 und 9 zu einer Messkammer 14 erweitert, wobei die beispielsweise kreisförmige Grundfläche der Messkammer 14 einen optischen Sensor 15 aufweist. Der optische Sensor 15 kann beispielsweise in einer Indikatorschicht 16 einen Indikator zur Messung des $pO_2$, des $pCO_2$ oder des pH-Wertes der Vollblutprobe aufweisen und mit einer analytpermeablen Deckschicht versehen sein, welche gleichzeitig als rote Kalibrierfläche 12 dient. Der Kapillarkanal 1 mit der Messkammer 14 ist in der Ausführungsvariante gemäß Fig. 4 und 5 in einer Einwegkassette 17 angeordnet, welche zweiteilig (Teile 18 und 19) aufgebaut ist und in ein hier nicht weiter dargestelltes Auswertegerät einbringbar ist, welches über die optischen Einrichtungen 2 und 3 verfügt. Aus Fig. 4 ist der Überlappungsbereich 11 des Anregungslichtkegels 8 mit dem Detektionskegel 9 erkennbar.

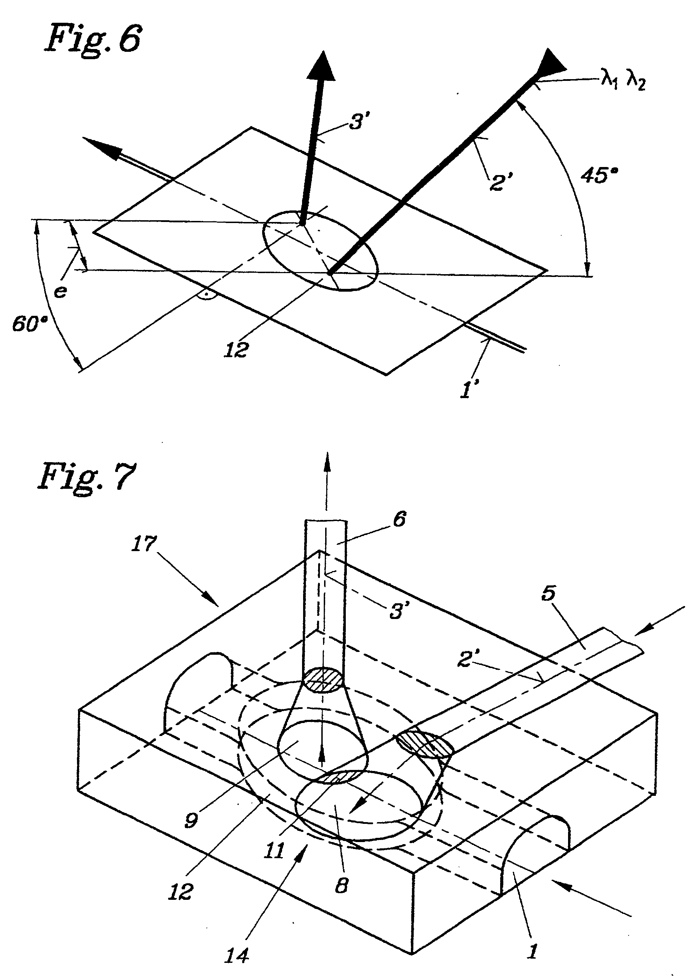

Die Fig. 6 und 7 zeigen die Messanordnung gemäß Fig. 4 und 5 in dreidimensionaler Darstellung, wobei Fig. 6 auf die rein geometrischen Gegebenheiten abgestellt ist. Aus Fig. 7 ist ein flacher Kapillarkanal ersichtlich, welcher sich strömungsgünstig in die Messkammer 14 erweitert. Die Höhe des Kapillarkanals und der Messkammer beträgt ungefähr 0,7 mm, die Eindringtiefe der verwendeten Messstrahlung bei mit Vollblut gefüllter Kapillare ca. 0,3 mm.

Bei der Kalibrierung wird das Anregungslicht über den Lichtleiter 5 in die Kassette 17 eingekoppelt und an der Kalibrierfläche 12 wellenlängenspezifisch reflektiert. Das reflektierte Licht kann von der Empfangsoptik detektiert werden, wobei sich herausgestellt hat, dass bei Verwendung von mindestens zwei Wellenlängen, bei welchen die Kalibrierfläche 12 unterschiedlich Absorptionswerte und damit auch unterschiedliche Reflexionswerte zeigt, eine Kalibriergröße angegeben werden kann, die den Ein- und Auskoppelverlusten proportional ist. Die Kalibriergröße kann z. B. aus dem Verhältnis der Reflexionswerte der beiden Messwellenlängen gebildet werden. Die Verhältniszahl muss in einem bestimmten, vorgegebenen Bereich liegen um die Kalibrierung positiv abzuschließen. Damit ist es möglich, kassettenspezifische Ankoppelverluste vor der Messung zu kalibrieren. Bei der Verwendung von mehr als zwei Messwellenlängen kann die Genauigkeit der Kalibrierung entsprechend verbessert werden.

Die Diagramme in den Fig. 8 und 9, bei welchen auf der Ordinate die Absorption A und auf der Abszisse die Sauerstoffsättigung $O_{2sat}$ in Prozent aufgetragen ist, zeigen, dass das Summensignal $A_1 + f.A_2$ der Absorptionswerte $A_1$ und $A_2$ bei den Wellenlängen $\lambda_1 = 780$ nm und $\lambda_2 = 850$ nm für unterschiedliche Werte von $O_{2sat}$ konstant ist. In Fig. 8 beträgt tHb = 160mg/ml in Fig. 9 tHb = 220mg/ml. Der Faktor f = 1.13.

**Patentansprüche**

1. Messanordnung zur optischen Bestimmung der totalen Hämoglobinkonzentration (tHb) in nicht hämolysiertem Vollblut, mit einem Kapillarkanal (1) zur Aufnahme einer Blutprobe, einer Anregungseinrichtung (2) zur Einstrahlung von zumindest zwei Messwellenlängen ($\lambda_1$ und $\lambda_2$) und einer auf der selben Seite der Kapillare angeordneten Detektionseinrichtung (3), wobei die optischen Achsen (2', 3') der Anregungseinrichtung (2) und der Detektionseinrichtung (3) auf den Kapillarkanal (1) gerichtet sind, und die Anregungseinrichtung (2) einen durch einen Strahlöffnungswinkel $\alpha$ definierten, von der Anregungseinrichtung ausgehenden Anregungslichtkegel (8) und die Detektionseinrichtung (3) einen durch einen Akzeptanzwinkel $\beta$ definierten, von der Detektionseinrichtung ausgehenden Detektionskegel (9) aufweist, **dadurch gekennzeichnet, dass** die Neigungswinkel ($\beta_1$, $\beta_2$) der optischen Achsen (2', 3') und deren Abstand (e) an der Schnittstelle mit der Kapillare (1) derart auf den Kapillardurchmesser abgestimmt sind, dass sich der Anregungslichtkegel (8) und der Detektionskegel (9) während einer Kalibriermessung bei mit wässriger Lösung gefülltem Kapillarkanal (1) an der der Anregungseinrichtung und der Detektionseinrichtung gegenüberliegenden Innenwand (10) des Kapillarkanals (1) überlappen und während einer Messung bei mit Vollblut gefülltem Kapillarkanal (1), bedingt durch die verringerte mittlere Eindringtiefe der Messstrahlung, nicht überlappen, so dass die Messung durch Streuung an den Blutzellen erfolgt.

**2.** Messanordnung nach Anspruch 1 **dadurch gekennzeichnet, dass** die Messanordnung eine erste Messwellenlänge mit $\lambda_1 < 805$ nm und eine zweite Messwellenlänge mit $\lambda_2 > 805$ nm bereit stellt, derart, dass für die Absorptionskoeffizienten $\sigma O(\lambda)$ und $\sigma R(\lambda)$ der Hämoglobinderivate $O_2Hb$ und RHb bei den beiden Messwellenlängen $\lambda_1$ und $\lambda_2$ gilt: $\sigma O(\lambda_1)$ ungefähr gleich $\sigma R(\lambda_2)$ und $\sigma R(\lambda_1)$ ungefähr gleich $\sigma O(\lambda_2)$.

**3.** Messanordnung nach Anspruch 2, **dadurch gekennzeichnet, dass** $\lambda_1$ zwischen 780 und 790 nm, vorzugsweise bei $785 \pm 3$ nm liegt, sowie dass $\lambda_2$ zwischen 830 und 850 nm, vorzugsweise bei $836 \pm 3$ nm, liegt.

**4.** Messanordnung nach einem der Ansprüche 1 bis 3, **dadurch gekennzeichnet, dass** die Innenwand (10) des Kapillarkanals (1) zumindest im Bereich (11) des sich mit dem Anregungslichtkegel (8) überlappenden Detektionskegel (9) eine rote Kalibrierfläche (12) aufweist.

**5.** Messanordnung nach Anspruch 4, **dadurch gekennzeichnet, dass** die rote Kalibrierfläche (12) einen Mantelbereich der Innenfläche (10) des Kapillarkanals (1) bedeckt, welcher sich über ca. 20 bis 40% des Umfangs erstreckt und der Anregungs- und Detektionseinrichtung (2, 3) gegenüberliegt.

**6.** Messanordnung nach Anspruch 4, **dadurch gekennzeichnet, dass** sich der Kapillarkanal (1) im Bereich (11) der sich überlappenden Anregungslicht- bzw. Detektionskegel (8, 9) zu einer Messkammer (14) erweitert, wobei eine Wand der Messkammer (14) einen optischen Sensor (15) aufweist, dessen für den zu messenden Analyten permeable Deckschicht gleichzeitig als rote Kalibrierfläche (12) dient.

**7.** Messanordnung nach Anspruch 6, **dadurch gekennzeichnet, dass** der optische Sensor (15) eine Indikatorschicht (16) mit einem Indikator zur Messung des $pO_2$, des $pCO_2$ oder des pH-Wertes der Vollblutprobe aufweist.

**8.** Messanordnung nach einem der Ansprüche 1 bis 7, **dadurch gekennzeichnet, dass** der Kapillarkanal (1) in einer Einwegkassette (17) angeordnet ist, welche in ein die Anregungsund Detektionseinrichtung (2, 3) aufweisendes Auswertegerät einbringbar ist.

**Claims**

**1.** Measuring apparatus for optically determining the total haemoglobin concentration (tHb) in non-haemolyzed whole blood, comprising a capillary channel (1) for holding a blood sample, and an excitation device (2) for supplying at least two measurement wavelengths ($\lambda_1$ and $\lambda_2$), and a detection device (3) located on the same side of the capillary, where the optical axes (2', 3') of the excitation device (2) and the detection device (3) are directed towards the capillary channel (1), and the excitation device (2) has an excitation light cone (8) defined by an opening angle $\alpha$ and departing from said excitation device, and the detection device (3) has a detection cone (9) defined by an acceptance angle $\beta$ and departing from said detection device, **characterized in that** the inclination angles ($\beta_1$, $\beta_2$) of optical axes (2', 3') and the distance e of said axes at the intersection points with the capillary channel (1) are chosen with respect to the capillary diameter such that the excitation light cone (8) and the detection cone (9) overlap on the inner wall (10) of the capillary channel (1), which is situated opposite of said excitation device and said detection device, if said capillary channel (1) is filled with an aqueous solution during a calibration measurement, and that they do not overlap if said capillary channel (1) is filled with whole blood during measuring on account of the reduced mean penetration depth of the measurement radiation, such that the light scattered by the blood cells is measured.

**2.** Measuring apparatus according to claim 1, **characterized in that** a first measurement wavelength of $\lambda_1 < 805$ nm and a second measurement wavelength of $\lambda_2 > 805$ nm are supplied by said apparatus in such a way that for absorption coefficients $\sigma O(\lambda)$ and $\sigma R(\lambda)$ of the haemoglobin derivatives $O_2Hb$ and RHb $\sigma O(\lambda_1)$ will approximately equal $\sigma R(\lambda_2)$ and $\sigma R(\lambda_1)$ will approximately equal $\sigma O(\lambda_2)$ at both wavelengths $\lambda_1$ and $\lambda_2$.

**3.** Measuring apparatus according to claim 2, **characterized in that** $\lambda_1$ is between 780 and 790 nm, and preferably at $785 \pm 3$ nm, and $\lambda_2$ is between 830 and 850 nm, and preferably at $836 \pm 3$ nm.

**4.** Measuring apparatus according to any of claims 1 to 3, **characterized in that** the inner wall (10) of the capillary channel (1) is provided with a red calibration surface (12), at least in the region (11) of the overlapping detection light cone (9) and excitation light cone (8), respectively.

5. Measuring apparatus according to claim 4, **characterized in that** the red calibration surface (12) covers an area on the inner wall (10) of the capillary channel (1), which extends over some 20 to 40 percent of the circumference and is situated opposite of the excitation and detection device (2, 3).

6. Measuring apparatus according to claim 4, **characterized in that** the capillary channel (1) is enlarged to form a measuring chamber (14) in the region (11) of the overlapping excitation light and detection cones (8, 9), one wall of said measuring chamber (14) being provided with an optical sensor (15) whose cover layer, which is permeable to the analyte be measured, is configured as a red calibration surface (12).

7. Measuring apparatus according to claim 6, **characterized in that** the optical sensor (15) exhibits an indicator layer (16) containing an indicator for $pO_2$, $pCO_2$ or pH measurement in the whole blood sample.

8. Measuring apparatus according to any of claims 1 to 7, **characterized in that** the capillary channel 1 is positioned in a oneway cartridge (17) that can be inserted into an evaluation unit including the excitation and detection device (2, 3).

**Revendications**

1. Dispositif de mesure pour la détermination optique de la concentration totale en hémoglobine (tHb) dans un sang complet non hémolysé, avec un canal capillaire (1) pour recevoir un échantillon de sang, un dispositif d'excitation (2) pour émettre un rayonnement au moins à deux longueurs d'onde de mesure ($\lambda_1$ et $\lambda_2$) et un dispositif de détection (3) agencé sur le même côté du capillaire, les axes optiques (2', 3') du dispositif d'excitation (2) et du dispositif de détection (3) étant dirigés sur le canal capillaire (1), le dispositif d'excitation (2) présentant un cône (8) de lumière excitatrice partant du dispositif d'excitation et défini par un angle d'ouverture $\alpha$ du faisceau, et le dispositif de détection (3) présentant un cône de détection (9) partant du dispositif de détection et défini par un angle de réception $\beta$, **caractérisé en ce que** les angles d'inclinaison ($\beta_1$, $\beta_2$) des axes optiques (2', 3') et leur distance mutuelle (e) aux points d'intersection avec le capillaire (1) sont choisis en fonction du diamètre du capillaire de manière à ce que le cône de la lumière excitatrice (8) et le cône de détection (9), lors d'une mesure de calibrage avec un canal capillaire (1) rempli d'une solution aqueuse, se chevauchent au niveau de la paroi interne (10) du canal capillaire (1) faisant face au dispositif d'excitation et au dispositif de détection et **en ce que**, lors d'une mesure avec le canal capillaire (1) rempli de sang complet et, consécutivement, avec une profondeur de pénétration moyenne moindre du rayonnement de mesure, ces cônes ne se chevauchent pas, ce qui permet une mesure basée sur la dispersion par les globules rouges.

2. Dispositif de mesure selon la revendication 1, **caractérisé en ce que** la mesure avec une première longueur d'onde de mesure $\lambda_1 < 805$ nm et avec une seconde longueur d'onde de mesure $\lambda_2 > 805$ nm se fait de manière à ce que les coefficients d'absorption $\sigma O(\lambda)$ et $\sigma R(\lambda)$ des dérivés $O_2Hb$ et RHb de l'hémoglobine aux deux longueurs d'onde de mesure $\lambda_1$ et $\lambda_2$ soient comme suit: $\sigma O(\lambda_1)$ approximativement égal à $\sigma R(\lambda_2)$ et $\sigma R(\lambda_1)$ approximativement égal à $\sigma O(\lambda_2)$.

3. Dispositif de mesure selon la revendication 2, **caractérisé en ce que** $\lambda_1$ est entre 780 et 790 nm et, de préférence, à $785 \pm 3$ nm et que $\lambda_2$ est entre 830 et 850 nm et, de préférence, à $836 \pm 3$ nm.

4. Dispositif de mesure selon l'une des revendications 1 à 3, **caractérisé en ce que** la paroi interne (10) du canal capillaire (1) présente, au moins dans la région de chevauchement (11) du cône de lumière excitatrice (8) et du cône de détection (9), une surface rouge de calibrage (12).

5. Dispositif de mesure selon la revendication 4, **caractérisé en ce que** la surface rouge de calibrage (12) couvre une région convexe de la surface interne (10) du canal capillaire (1), qui s'étend sur plus de 20 à 40 % environ de la circonférence et qui se trouve à l'opposé des dispositifs d'excitation et de détection (2, 3)

6. Dispositif de mesure selon la revendication 4, **caractérisé en ce que** le canal capillaire (1) dans la région de chevauchement (11) du cône de lumière excitatrice et du cône de détection (8, 9), s'élargit pour former une chambre de mesure (14), où une paroi de la chambre de mesure (14) porte une sonde optique (15), dont la couche de revêtement, perméable à l'analyte à mesurer, sert en même temps de surface rouge de calibrage (12).

7. Dispositif de mesure selon la revendication 6, **caractérisé en ce que** la sonde optique (15) présente une couche indicatrice (16), avec un indicateur pour mesurer la $pO_2$, la $pCO_2$ ou la valeur du pH de l'échantillon de sang complet.

8. Dispositif de mesure selon l'une des revendications 1 à 7, **caractérisé en ce que** le canal capillaire (1) est agencé dans une cassette à usage unique (17) qui peut être introduite dans un appareil de mesure comprenant le dispositif d'excitation et le dispositif de détection (2, 3).

*Fig.1*

*Fig.2*

*Fig.3*

*Fig. 4*

60°

*Fig. 5*

$\beta_1=45°$

## Fig.6

## Fig.7

## Fig.8

### tHb=160mg/ml

## Fig.9

### tHb=220mg/ml